# EUROPÄISCHE PATENTANMELDUNG

(11) **EP 1 438 932 A1**
(43) Veröffentlichungstag der Anmeldung: **21.07.2004**
(21) Anmeldenummer: 03001041.7
(22) Anmeldetag: 17.01.2003
(51) Int. Cl.: A61F 2/36, A61F 2/32, A61F 2/30

(54) **Hüftprothese mit einem in den Oberschenkelknochen einzusetzenden Schaft**

(71) Anmelder: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(57) **Zusammenfassung**

Hüftprothese mit einem in den Oberschenkelknochen einzusetzenden Schaft (1), dessen proximaler, im metaphysären Bereich des Oberschenkelknochens einzusetzender Teil (6) auf der Vorder- und Rückseite mindestens je eine vorspringende Rippe (8) mit nach medial steiler Flanke (23) aufweist. Diese Rippe (8) hat zumindest in einem Teil ihres Verlaufs eine von der Längsrichtung (27) des Schafts (1) abweichende, sich nach oben dem Schenkelhals (2) zuneigende Richtung hat. Dadurch wird beim Eintreiben des Schafts in den Knochen die in der Umgebung der Rippe (8) befindliche Knochensubstanz verdichtet und zu höherer Kraftaufnahme befähigt.

## Beschreibung

Für die Verankerung eines Prothesenschaftes im Oberschenkelknochen liegen im metaphysären Bereich andere Bedingungen als im diaphysären Bereich vor. Im metaphysären Bereich, d.h. im wesentlichen im Bereich oberhalb des kleinen Trochanters, ist der weiträumige Knochen von spongiöser Knochensubstanz gefüllt, in welcher für die Aufnahme eines Prothesenschafts ein künstlicher Aufnahmekanal geschaffen werden muß. Da die spongiöse Knochensubstanz weich ist, sind die Kraftübertragungsmöglichkeiten in diesem Bereich begrenzt. Unterhalb des kleinen Trochanters ist der engere Markraum von kräftiger Knochenrinde begrenzt, die für die Kraftübertragung wesentlich bessere Voraussetzungen bietet. Es wurden daher Prothesenschäfte entwickelt, die speziell zur Verankerung und Verkeilung in diesem diaphysären Bereich des Knochens geeignet sind (Schneider: Die Totalprothese der Hüfte, Seite 214 ff.). Ihre verlässliche Verankerung im diaphysären Knochenbereich hat die Folge, daß der metaphysäre Knochenbereich entlastet wird. Wenn Knochensubstanz nicht belastet wird, wird sie allmählich abgebaut. Dies ist unerwünscht. Die Erfindung versucht daher bei einem diaphysär verankerten Prothesenschaft Kraftübertragungsmöglichkeiten im metaphysären Bereich zu schaffen, die dem Knochenabbau entgegenwirken.

Die erfindungsgemäße Lösung liegt in den Merkmalen des Anspruchs 1 und vorzugsweise denjenigen der Unteransprüche.

Demnach ist vorgesehen, daß auf den Vorder- und Rückseiten des proximalen, im metaphysären Bereich des Oberschenkelknochens einzusetzenden Teils des Prothesenschafts mindestens je eine vorspringende Rippe mit nach medial steiler Flanke vorgesehen ist, die mindestens in einem Teil ihres Verlaufs eine von der Längsrichtung des Schafts abweichende, sich nach oben dem Schenkelhals zuneigende Richtung hat. Diese Form hat den Vorteil, daß beim Einsetzen der Prothese die mediale Flanke der Rippe wie ein Keil die in Einschubrichtung vor ihr befindliche, spongiöse Knochensubstanz verdichtet, so daß sie zu stärkerer Kraftübertragung befähigt wird. Diese Wirkung wird vornehmlich dann erzeugt, wenn der Winkel, den diese Rippenflanke mit der Längsrichtung des Schafts einschließt, zwischen 5 und 15°, vorzugsweise bei etwa 10° liegt.

Eine entsprechende Kompression auf den anterioren bzw. posterioren Seiten der Rippen kann dadurch erfindungsgemäß erzielt werden, daß die Höhe der Rippen über der jeweiligen Oberfläche des Schaftgrundkörpers von unten nach oben zunimmt. Die Rippe ist somit in doppelter Richtung keilförmig, nämlich erstens zur medialen Seite hin und zweitens zur anterioren bzw. posterioren Seite hin. Nach lateral zu, d.h. auf der der steilen Flanke abgewendeten Seite, kann die Höhe der Rippe allmählich abnehmen.

Die Kompression der spongiösen Knochensubstanz durch die Rippen setzt voraus, daß an der Stelle, an der sich nach der Implantation die Rippen befinden, zuvor Knochensubstanz vorhanden war. Wenn vor dem Einsetzen der Prothese im metaphysären Bereich des Knochens ein Aufnahmekanal für den Prothesenschaft künstlich gebildet wird, soll dieser nur der Querschnittsform des Grundkörpers des Prothesenschafts entsprechen und also noch keine Ausbuchtungen für die spätere Aufnahme der Rippen bilden. Wenn eine Raspel zur Formung dieses Aufnahmekanals verwendet wird, soll sie in ihrer Form also nur dem Schaftgrundkörper entsprechen, ohne Einrichtungen zur Materialabnahme im Rippenbereich aufzuweisen. Alternativ besteht auch die Möglichkeit die Raspel mit Rippen auszurüsten, die den Rippen des Prothesenschaft entsprechen und ohne Materialabnahme zur Kompression der Knochensubstanz eingerichtet sind. Die weiter oben beschriebene Kompression der Knochensubstanz im Kraftübertragungsbereich der Rippen kommt dann schon durch die Raspel zustande oder wird von dieser teilweise vorbereitet.

Die Erfindung wird im folgenden näher unter Bezugnahme auf die Zeichnung erläutert, die ein vorteilhaftes Ausführungsbeispiel veranschaulicht. Es zeigen:
- Fig. 1: eine Ansicht von vorne;
- Fig. 2: eine Ansicht von medial;
- Fig. 3: eine Ansicht von oben; und
- Fig. 4 bis 6: Schnitte durch den Schaft an den entsprechenden Höhen desselben.

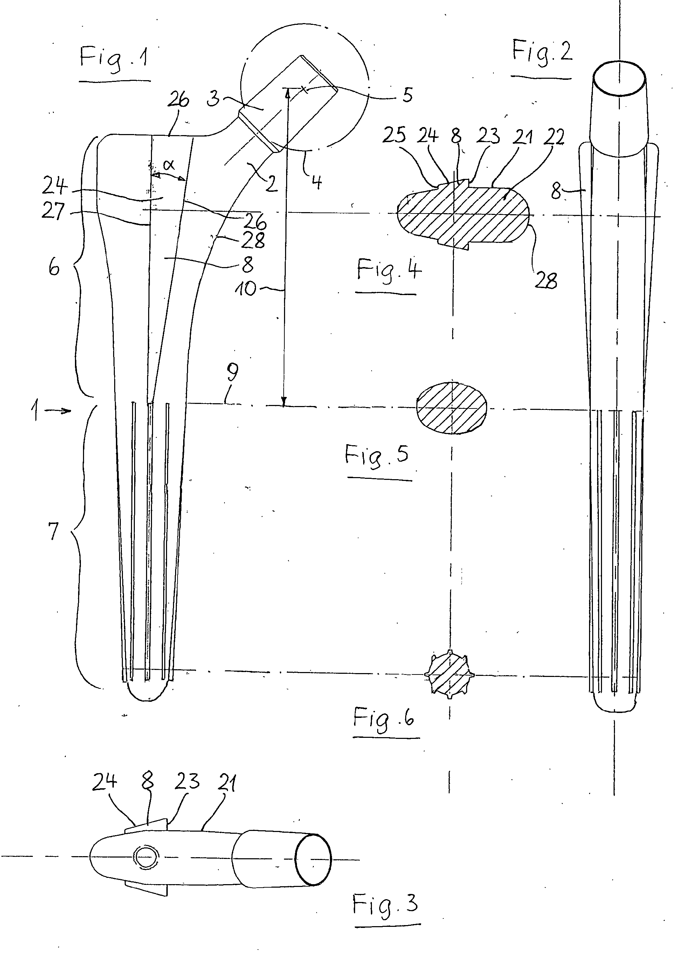

Die Prothese besteht aus einem Schaft 1, einem Hals 2 und einem Konus 3 zum Aufsetzen eines Gelenkkopfs 4, dessen Umfang strichpunktiert angedeutet ist. Der Schaft setzt sich zusammen aus einem proximalen Abschnitt 6 und einem distalen Abschnitt 7. Der proximale Abschnitt ist im Querschnitt langgestreckt in LM-Richtung, wie Fig. 4 und 5 es zeigen. Er ist mit einem Rippenpaar 8 versehen zur Kraftübertragung auf die im epiphysären Bereich des Oberschenkelknochens den Schaft umgebende, spongiöse Knochensubstanz.

Die Übergangsstelle 9 zwischen dem proximalen und dem distalen Abschnitt des Schafts ist so angeordnet, daß sie im implantierten Zustand etwa beim kleinen Trochanter, vorzugsweise ein wenig unterhalb desselben, zu liegen kommt und der distale Schaftabschnitt 7 demzufolge in einem Bereich des Markkanals liegt, in welchem dieser durch eine starke Knochenrinde begrenzt ist. Die Übergangsstelle braucht an der Prothese nicht besonders markiert zu sein. Sie ist dadurch bestimmt, daß sie an der Stelle liegt, an der im implantierten Zustand der kleine Trochanter bzw. vorzugsweise dessen Unterkante anzunehmen ist. Sie liegt in der Regel etwa 7 bis 9 cm tiefer als der Mittelpunkt 5 des Gelenkkopfes 4, gemessen gemäß Pfeil 10 in Schaftrichtung.

Der distale Schaftabschnitt ist so ausgebildet, daß er zur Verankerung im diaphysären Bereich des Oberschenkelknochens geeignet ist. Zur Erzielung eines festen Sitzes ist sein Schaftkern schwach konisch und mit Längsrippen besetzt. Dadurch wird Knochensubstanz, die sich im Zwischenraum zwischen der Oberfläche des Schaftkerns 15 und der kortikalen Markraumbegrenzung befindet, komprimiert, wobei sie durch die Rippen festgehalten wird. Der distale Abschnitt kann auch in anderer Weise so ausgebildet sein, daß er zur primären Verankerung des Schafts in der Diaphyse des Knochens geeignet ist.

Die Rippen 8 erheben sich von den anterioren und posterioren Oberflächen 21 des Schaftgrundkörpers 22. Sie weisen eine nach medial gerichtete steile Flanke 23 und einen anterioren bzw. posterioren Oberflächenabschnitt 24 auf, der lateral durch eine Kante 25 begrenzt ist. Die Oberfläche 24 kann sich mit zunehmender Entfernung von der Flanke 23 zu der Oberfläche 21 hin absenken, so daß die Rippe eine etwa dreieckige oder trapezförmige Querschnittsgestalt erhält, wie man dies in Fig. 3 und 4 erkennt.

Die Rippen 8 beginnen am Übergang 9 zwischen dem proximalen Abschnitt und dem distalen Abschnitt 7 des Schafts mit der Höhe Null und geringer Breite. Nach oben hin wachsen sie gleichmäßig zu ihrer maximalen Höhe und Breite, die sie am oberen Ende 26 erreichen. Mit der Längsachse 27 des Schafts schließt die Flanke 23 einen Winkel α ein, der im Ausführungsbeispiel bei etwa 8° liegt. Die Höhe der Flanke 23 am oberen Ende 26 des Schafts liegt zwischen 2 und 4 mm, vorzugsweise bei etwa 3 mm.

Die Querschnittsfläche der Rippen vergrößert sich somit von unten nach oben keilförmig in doppelter Richtung, nämlich zu der Flanke 23 und zu den anterioren bzw. posterioren Flächen 24 hin. Wenn die für die Aufnahme des Prothesenschafts im metaphysären, spongiösen Bereich des Oberschenkelknochens geformte Höhlung im Querschnitt dem Grundkörper 22 des Schafts gleicht, verdrängen die Rippen 8 beim Eintreiben des Schafts in diese Höhlung das dort befindliche spongiöse Material und verdichten es. Es wird dadurch geeigneter zur Kraftübertragung.

Das Ausführungsbeispiel zeigt einen linearen Verlauf der Rippen 8. Ihre Keilform kann aber auch einen nicht linearen Verlauf haben.

Wenngleich die Prothese zur primären Verankerung in der Diaphyse bestimmt ist, tragen die Rippen 21 ebenso wie die übrigen nach medial gerichteten Flächen 28 des Prothesenschafts im metaphysären Bereich zur Kraftübertragung bei. Die Metaphyse des Knochens wird dadurch an der Kraftübertragung beteiligt. Die Gefahr ihrer Rückbildung wird dadurch vermindert. Der langzeitig sichere Halt der Prothese im Knochen wird verbessert.

## Patentansprüche

1. Hüftprothese mit einem in den Oberschenkelknochen einzusetzenden Schaft (1), dessen proximaler, im metaphysären Bereich des Oberschenkelknochens einzusetzender Teil (6) auf der Vorder- und Rückseite mindestens je eine vorspringende Rippe (8) mit nach medial steiler Flanke (23) aufweist, **dadurch gekennzeichnet, daß** diese Rippe (8) zumindest in einem Teil ihres Verlaufs eine von der Längsrichtung (27) des Schafts (1) abweichende, sich nach oben dem Schenkelhals (2) zuneigende Richtung hat.

2. Prothese nach Anspruch 1, **dadurch gekennzeichnet, daß** die Rippe (8) geradlinig unter einem Winkel (Alpha) von 5 bis 15° gegenüber der Längsrichtung (27) des Schafts (1) verläuft.

3. Prothese nach Anspruch 1 oder 2, **dadurch gekennzeichnet, daß** die Höhe der Rippe (8) über der Oberfläche (21) des Schaftgrundkörpers (22) von unten nach oben zunimmt.

4. Prothese nach einem der Ansprüche 1 bis 3, **dadurch gekennzeichnet, daß** die Höhe der Rippe (8) von der die steile Flanke (23) begrenzenden Kante nach lateral abnimmt.

5. Prothese nach einem der Ansprüche 1 bis 4, **dadurch gekennzeichnet, daß** sie für primär diaphysäre Verankerung ausgebildet ist.
